# EUROPEAN PATENT APPLICATION

(11) **EP 2 168 587 A1**
(43) Date of publication of application: **31.03.2010**
(21) Application number: 08715058.7
(22) Date of filing: 27.03.2008
(51) Int. Cl.: A61K 36/185, A61P 3/02, A61K 131/00

(54) **A METHOD FOR PREPARING A MIXTURE CONTAINING NATURAL VITAMIN P FROM HIPPOPHAE RHAMNOIDES L.**

(30) Priority: 13.06.2007 CN 200710018178
(71) Applicant: Qinghai Tsinghua Biotry Bio-tech Co., Ltd, Qinghai 810016 (CN)
(72) Inventor: LU, Changzheng, Qinghai 810016 (CN); LI, Shuzhi, Qinghai 810016 (CN); SHAN, Yongkai, Qinghai 810016 (CN); ZENG, Duanguo, Qinghai 810016 (CN); XIE, Qing, Qinghai 810016 (CN)
(74) Representative: Hatzmann, Martin
(86) International application number: PCT/CN2008/000613
(87) International publication number: WO 2008/151501

(57) **Abstract**

The present invention provides a method for preparing a mixture containing natural vitamin P from Hippophae rhamnoides L. The mixture containing 0.5 - 20% of vitamin P and 2 - 30% of vitamin C are prepared by taking juice, degassing, enzymolysis, degreasing, homogenizing, deacidifying, desugaring, microfiltrating, freeze-drying and pulverizing. The method is simple, a combination of low-temperature and biophysics applied in the method solves the problem of brown change of high active substances in Hippophae rhamnoides L caused by high-temperature oxidation; during the entire production process, no organic solvent is required; no residue remains; the cost is low; and the yield of bioactive substances is high; the co-existing of vitamin P and vitamin C in the prepared products can promote a better absortion in human body.

## Description

### TECHNICAL FIELD

This invention relates to a method for preparing natural active substances from plant fruit, specifically, relates to a method for preparing a mixture containing natural vitamin P from Hippophae rhamnoides L.

### BACKGROUND ART

Seabuckthorn fruit, also known as Elaeagnus, Hippophae fruit, Black thorn, Acid thorns, Qicharigana (Mongolian name), Dapu (Tibetan name), Jihan (Uighur name), is the ripening fruit of Hippophae rhamnoides L belonging to Elaeagnaceae plant. Hippophae rhamnoides L. is an abundant resource of vitamin P (polyphenols and flavonoids), it can enhance the durability of the human body, reduce the permeability of capillary wall, protect vitamin C in the body from damage and promote the absorption of vitamin C.

At present, there are vitamin C, flavonoids and other monomer products extracted from Hippophae rhamnoides L, but there remains no mixture containing vitamin P prepared from Hippophae rhamnoides L. Also, in the existing preparation methods, traditional methods, such as water vapor distillation, vacuum distillation, solvent extraction and so on, are mostly used, said methods render high costs and low yields, the obtained products are easily oxidized, and the solvents used for extraction can not be removed completely.

### CONTENTS OF INVENTION

The object of the invention is to overcome the shortcomings in the prior art and to provide a method for preparing a mixture containing natural vitamin P from Hippophae rhamnoides L.

The other object of the invention is to provide a mixture that contains natural vitamin P.

The method for preparing a mixture containing natural vitamin P from Hippophae rhamnoides L comprises the following steps:
(1) Taking juice: seabuckthorn fruits are washed and then centrifuged at 3500 - 4500 r / min rotational speed for 5 to 10 minutes to obtain seabuckthorn juice;
(2) Degassing: the above seabuckthorn juice is degassed under the conditions of a vacuum degree of 5.13 - 5.33Pa and temperature between 41 - 43 °C until no obvious air bubbles are produced in the mixing state;
(3) Enzymolysis: to the degassed seabuckthorn juice is added 95% hydroxide solution to adjust pH to 3 - 4, the pectinase is added to an enzymolysis reactor with membrane separation with an amount of 2 - 4 g /1 to perform enzymolysis for 2 to 6 hours;
(4) Degreasing: the seabuckthorn juice after enzymolysis is introduced into a disc-type degreasing machine, its fat content is controlled within a range of 0.2-0.5%;
(5) Homogenizing: the seabuckthorn juice after degreasing is homogenized two times with high pressure homogenizer at temperature between 26 and 40°C, the first homogeneity is carried out at 14 - 16.4MPa pressure for 3 - 5 hours, the second homogeneity is carried out at 9 - 11.8MPa pressure for 0.5 - 3 hours;
(6) Deacidifying: then calcium carbonate is added with 1 - 5% by weight of the seabuckthorn juice, after going through ion exchange resin, lactobacillus with the concentration of 10 - 25ppm is added to reduce the acidity to 7 - 10 g /1;
(7) Desugaring: the seabuckthorn juice is heated to 20 - 30°C, glucose oxidase is added with 0.005 - 0.015%, then 5 - 10% hydrogen peroxide is added at the rate of 0.2 - 0.5% per hour to desugar, the reaction time is 1 - 3 hours;
(8) Microfiltrating: the filtration is carried out using cellulose acetate membrane under the operation pressure of 3 - 14 MPa;
(9) Freeze-drying: after the vacuum degree reaches 22 - 26 Pa, vacuumizing will be continued for 1 to 2 hours, when the temperature is adjusted to -40 - -45°C, the seabuckthorn juice is freeze-dried for 15 to 26 hours;
(10) Pulverizing: the freeze-dried mixture containing vitamin P is pulverized using low temperature pulverizer at -20 - -30°C for 2 - 8 minutes, the fineness of pulverized mixture reaches between 38 - 58µ m. Wherein, the said degassing is preferably carried out under the conditions of a vacuum degree of 5.2 - 5.3Pa and temperature 42°C.

The said ion exchange resin refers to anion exchange resin, specifically refers to a mixed resin with D-92 macroporous weakly basic styrene type anion exchange resin, D-60C isoporous & monosphere gel-type styrene series strong acid resin and D-A21 alcohol refined dedicated macroporous absorption resin at a 1:1:1 ratio or a mixed resin with D-92 macroporous weakly basic styrene type anion exchange resin, D • 70A isoporous & monosphere gel-type styrene series strong alkali resin, D-66 alcohol refined dedicated macroporoous strong acid resin at a 1:1:1 ratio.

The said lactobacillus refers to Lactobacillus lactislactobacillus, leuconostoc, pediococcus or other commonly used lactobacillus.

The said acidity is calculated based on the amount of "tartaric acid".

The said microfiltrating is preferably operated using cellulose acetate membrane at the pressure of 8 - 10 MPa;

The pore size of said cellulose acetate membrane is within 0.3 - 0.7µ m. The said freeze-drying is preferably carried out by continuing to vacuumize for 1.5 hours after the vacuum degree reaches 24 - 25 Pa, and then freeze-drying the seabuckthorn juice for 18 to 20 hours when the temperature is adjusted to -42 - -43°C;

The said pulverizing is preferably carried out by pulverizing the freeze-dried mixture containing vitamin P using low temperature pulverizer at -25 - -28°C for 5 minutes till the fineness of pulverized mixture reaches 45 µm.

The percentage described in the invention refers to weight percentage.

The natural vitamin P-contained mixture, obtained by using this invention, comprises vitamin P, vitamin C and fruit powder.

Wherein, preferably, the natural vitamin P-contained mixture comprises the following: 0.5 - 20% of vitamin P, 2 - 30% of vitamin C, the remaining being fruit powder.

The said vitamin P contains 60 - 80% of total flavonoids, 12 - 30% of anthocyanin and 3 - 20% of catechin.

The invention has the following advantages: the method for preparing the products is simple; using a combination of low-temperature and biophysics renders low cost and produces no residue; and it fundamentally solves the problem of brown change of high active substances in Hippophae rhamnoides L. caused by high-temperature oxidation; during the entire production process, no organic solvent is required; the yield of bioactive substances is high; the co-existing of vitamin P and vitamin C in the prepared products can promote the human body to fully absorb the nutrients, reduce fragility and permeability of blood vessels, enhance the activity of vitamin C, enhance immunity, anti-oxidation, and prevent cerebral hemorrhage, retinal hemorrhage, purpura and other diseases; also the prepared products can provide high-quality raw materials for high-grade natural medicines and health products.

### DESCRIPTION OF DRAWINGS

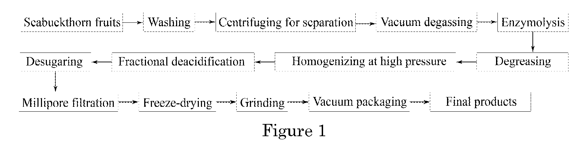

Figure 1 illustrates a flow chart of the method of preparing a mixture containing natural vitamin P from Hippophae rhamnoides L.

### MODE OF CARRYING OUT THE INVENTION

The following examples are provided to further illustrate the invention, not to be interpreted as limitations on the scope of the invention.

### Example 1

(1) Taking juice: 100kg fresh seabuckthorn fruits were washed and then centrifuged at 3500 r / min rotational speed for 10 minutes to obtain seabuckthorn juice;
(2) Degassing: 100kg above seabuckthorn juice was degassed under the conditions of a vacuum degree of 5.13 Pa and temperature of 43°C till no obvious air bubbles were produced in the mixing state;
(3) Enzymolysis: to the degassed seabuckthorn juice was added 95% sodium hydroxide solution to adjust pH to 3; pectinase was added to an enzymolysis reactor with membrane separation with an amount of 2 g / 1 to perform enzymolysis for 6 hours;
(4) Degreasing: the seabuckthorn juice after enzymolysis was introduced into a disc-type degreasing machine, its fat content was controlled at 0.2%;
(5) Homogenizing: the seabuckthorn juice after degreasing was homogenized twice with high pressure homogenizer at temperature of 26°C, the first homogeity was carried out at 14MPa pressure for 3 hours, and the second was carried out at 11.8MPa pressure for 3 hours;
(6) Deacidifying: then calcium carbonate was added with 1% by weight of the seabuckthorn juice, after going through ion exchange resin (D-92 macroporous weakly basic styrene type anion exchange resin, D •7OA isoporous & monosphere gel-type styrene series strong alkali resin, D-66 alcohol refined dedicated macroporous strong acid resin, mixed at a 1:1:1 ratio), Lactobacillus lactis with the concentration of 25ppm was added to reduce the acidity to 10 g / 1;
(7) Desugaring: the seabuckthorn juice was heated to 30°C, glucose oxidase was added with 0.015%, then 5% hydrogen peroxide was added at the rate of 0.5% per hour to desugar , the reaction time was 3 hours;
(8) Microfiltrating: the filtration was carried out using cellulose acetate membrane (0.3um) under the operation pressure of 3 MPa, allowing that the protein macro-molecules, impurities and bacteria can be entrapped, while soluble low molecular substances can go through the membrane along with the filtrate;
(9) Freeze-drying: after the vacuum degree reaches 22 Pa, vacuumizing was continued for 1 - 2 hours, then the temperature was adjusted to -40°C, the seabuckthorn juice was freeze-dried for 26 hours;
(10) Pulverizing: the freeze-dried mixture containing vitamin P was pulverized using low temperature pulverizer at -20°C for 8 minutes, the fineness of pulverized mixture reached 58um.
(11) Final product: 520g mixture containing 0.5% vitamin P (including 80% total flavonoids, 12 % anthocyanin and 8% catechin), 30% vitamin C and 69.5% fruit powder was prepared by vacuum packaging using aluminium foil.

### Example 2

(1) Taking juice: 100kg fresh seabuckthorn fruits were washed and then centrifuged at 4000 r / min rotational speed for 5 minutes to obtain seabuckthorn juice;
(2) Degassing: 100kg above seabuckthorn juice was degassed under the conditions of a vacuum degree of 5.33 Pa and temperature of 41°C till no obvious air bubbles were produced in the mixing state;
(3) Enzymolysis: to the degassed seabuckthorn juice was added 95% sodium hydroxide solution to adjust pH to 4; pectinase was added to an enzymolysis reactor with membrane separation with an amount of 4 g / 1 to perform enzymolysis for 2 hours;
(4) Degreasing: the seabuckthorn juice after enzymolysis was introduced into a disc-type degreasing machine, its fat content was controlled at 0.5%;
(5) Homogenizing: the seabuckthorn juice after degreasing was homogenized twice with high pressure homogenizer at temperature of 40°C, the first homogeity was carried out at 16.4MPa pressure for 5 hours, and the second was carried out at 9MPa pressure for 0.5 hour;
(6) Deacidifying: then calcium carbonate was added with 5% by weight of the seabuckthorn juice, after going through ion exchange resin (D-92 macroporous weakly basic styrene type anion exchange resin, D-60C isoporous & monosphere gel-type styrene series strong acid resin, D-A21 alcohol refined dedicated macroporous absorption resin, mixed at a 1:1:1 ratio), leuconostoc with the concentration of 10ppm was added to reduce the acidity to 7g / 1;
(7) Desugaring: the seabuckthorn juice was heated to 20°C, glucose oxidase was added with 0.005%, then 10% hydrogen peroxide was added at the rate of 0.2% per hour to desugar , the reaction time was 1 hour;
(8) Microfiltrating: the filtration was carried out using cellulose acetate membrane (0.7um) under the operation pressure of 14MPa, allowing that the protein macro-molecules, impurities and bacteria can be entrapped, while soluble low molecular substances can go through the membrane along with the filtrate;
(9) Freeze-drying: after the vacuum degree reaches 26 Pa, vacuumizing was continued for 1 hour, then the temperature was adjusted to -45°C, the seabuckthorn juice was freeze-dried for 15 hours;
(10) Pulverizing: the freeze-dried mixture containing vitamin P was pulverized using low temperature pulverizer at -30°C for 2 minutes, the fineness of pulverized mixture reached 45um.
(11) Final product: 500g mixture containing 20% vitamin P (including 60% total flavonoids, 37 % anthocyanin and 3% catechin), 2% vitamin C and 78% fruit powder was prepared by vacuum packaging using aluminium foil.

### Example 3

(1) Taking juice: 100kg fresh seabuckthorn fruits were washed and then centrifuged at 4500 r / min rotational speed for 8 minutes to obtain seabuckthorn juice;
(2) Degassing: 100kg above seabuckthorn juice was degassed under the conditions of a vacuum degree of 5.2 Pa and temperature of 42°C till no obvious air bubbles were produced in the mixing state;
(3) Enzymolysis: to the degassed seabuckthorn juice was added 95% sodium hydroxide solution to adjust pH to 3.5; pectinase was added to an enzymolysis reactor with membrane separation with an amount of 3 g / 1 to perform enzymolysis for 4h;
(4) Degreasing: the seabuckthorn juice after enzymolysis was introduced into a disc-type degreasing machine, its fat content was controlled at 0.35%;
(5) Homogenizing: the seabuckthorn juice after degreasing was homogenized twice with high pressure homogenizer at temperature of 35°C, the first homogeity was carried out at 15MPa pressure for 4 hours, and the second was carried out at 10.2MPa pressure for 2 hours;
(6) Deacidifying: then calcium carbonate was added with 3% by weight of the seabuckthorn juice, after going through ion exchange resin (D-92 macroporous weakly basic styrene type anion exchange resin, •7OA isoporous & monosphere gel-type styrene series strong alkali resin, D-66 alcohol refined dedicated macroporous strong acid resin mixed at a 1:1:1 ratio), pediococcus with the concentration of 18ppm was added to reduce the acidity to 8g / 1;
(7) Desugaring: the seabuckthorn juice was heated to 25°C, glucose oxidase was added with 0.01%, then 7.5% hydrogen peroxide was added at the rate of 0.35% per hour to desugar , the reaction time was 2 hours;
(8) Microfiltrating: the filtration was carried out using cellulose acetate membrane (0.5um) under the operation pressure of 8MPa, allowing that the protein macro-molecules, impurities and bacteria can be entrapped, while soluble low molecular substances can go through the membrane along with the filtrate;
(9) Freeze-drying: after the vacuum degree reaches 23.5 Pa, vacuumizing was continued for 1.4 hours, then the temperature was adjusted to -43°C, the seabuckthorn juice was freeze-dried for 20 hours;
(10) Pulverizing: the freeze-dried mixture containing vitamin P was pulverized using low temperature pulverizer at -25°C for 6 minutes, the fineness of pulverized mixture reached 45µm.
(11) Final product: 538g mixture containing 10% vitamin P (including 65% total flavonoids, 15% anthocyanin and 20% catechin), 20% vitamin C and 70% fruit powder was prepared by vacuum packaging using aluminium foil.

### INDUSTRIAL APPLICABILITY

The present invention provides a method for preparing a mixture containing natural vitamin P from Hippophae rhamnoides L. The mixtures containing 0.5 - 20% vitamin P and 2 - 30% vitamin C are prepared by taking juice, degassing, enzymolysis, degreasing, homogenizing, deacidifying, desugaring, microfiltrating, freeze-drying and pulverizing. The method is simple, a combination of low-temperature and biophysics used in the method solves the problem of brown change of high active substances in Hippophae rhamnoides L caused by high-temperature oxidation. During the entire production process, no organic solvent is required; no residue remains; the cost is low; and the yield of bioactive substances is high; the co-existing of vitamin P and vitamin C in the prepared products can promote the human body to better absorb the nutrients, reduce fragility and permeability of blood vessels, enhance the activity of vitamin C, enhance immunity, anti-oxidation, and prevent cerebral hemorrhage, retinal hemorrhage, purpura and other diseases, also the prepared products can provide high-quality raw materials for high-grade natural medicines and health products.

## Claims

1. A method for preparing a mixture containing natural vitamin P from Hippophae rhamnoides L, **characterized in that** it comprises the following steps:
(1)Taking juice: seabuckthorn fruits were washed and then centrifuged at 3500 - 4500 r/min rotational speed for 5 - 10 minutes to obtain seabuckthorn juice;
(2) Degassing: the above seabuckthorn juice is degassed under the conditions of a vacuum degree of 5.13 - 5.33Pa and temperature between 41 - 43°C till no obvious air bubbles were produced in the mixing state;
(3) Enzymolysis: to the degassed seabuckthorn juice is added 95% hydroxide solution to adjust pH to 3 - 4, the pectinase is added to an enzymolysis reactor with membrane separation with an amount of 2 - 4 g/l to perform enzymolysis for 2 - 6 hours;
(4) Degreasing: the seabuckthorn juice after enzymolysis is introduced into a disc-type degreasing machine, its fat content is controlled within the range of 0.2 - 0.5%;
(5) Homogenizing: the seabuckthorn juice after degreasing is homogenized twice with high pressure homogenizer at temperature between 26 - 40°C, the first homogeity is carried out at 14 - 16.4MPa pressure for 3 - 5 hours, the second homogeity is carried out at 9 - 11.8MPa pressure for 0.5 - 3 hours;
(6) Deacidifying: and then calcium carbonate is added with 1 - 5% by weight of the seabuckthorn juice, after going through ion exchange resin, lactobacillus with the concentration of 10 - 25ppm is added to reduce the acidity to 7 - 10 g /1;
(7) Desugaring: the seabuckthorn juice is heated to 20 - 30°C, glucose oxidase is added with 0.005 - 0.015%, then 5 - 10% hydrogen peroxide is added at the rate of 0.2 - 0.5% per hour to desugar, the reaction time is 1 - 3 hours;
(8) Microfiltrating: the filtration is carried out using cellulose acetate membrane under the operation pressure of 3 - 14 MPa;
(9) Freeze-drying: after the vacuum degree reaches 22 - 26 Pa, vacuumizing will be continued for 1 - 2 hours, when the temperature is adjusted to -40 - -45°C, the seabuckthorn juice is freeze-dried for 15 - 26 hours;
(10) Pulverizing: the freeze-dried mixture containing vitamin P is pulverized using low temperature pulverizer at -20 - -30°C for 2 - 8 minutes, the fineness of pulverized mixture reaches between 38 - 58µm.

2. The method as claimed in claim 1, **characterized in that** the said degassing means the above seabuckthorn juice is degassed under the conditions of a vacuum degree of 5.2 - 5.3Pa and temperature of 42°C.

3. The method as claimed in claim 1 or 2, **characterized in that** the said ion exchange resin refers to anion exchange resin.

4. The method as claimed in any of claims 1 to 3, **characterized in that** the pore size of said cellulose acetate membrane is within a range of 0.3 - 0.7µ m.

5. The method as claimed in any of claims 1 to 4, **characterized in that** said filtration is operated at the pressure of 8 - 10MPa.

6. The method as claimed in any of claims 1 to 5, **characterized in that** the said freeze-drying is carried out by continuing to vacuumize for 1.5 hours after the vacuum degree reaches 24 - 25 Pa, and then freeze-drying the seabuckthorn juice for 18 - 20 hours when the temperature is adjusted to -42 - -43°C.

7. The method as claimed in any of claims 1 to 6, **characterized in that** the said pulverizing is carried out by pulverizing the freeze-dried mixture containing vitamin P using low temperature pulverizer at -25 - -28°C for 5 minutes till the fineness of pulverized mixture reaches 45µm.

8. A Mixture prepared by the method as claimed in any of claims 1 to 7, **characterized in that** it contains vitamin P, vitamin C and fruit powder.

9. The mixture as claimed in claim 8, **characterized in that** it contains 0.5 - 20% of vitamin P, 2 - 30% of vitamin C, fruit powder being the remaining.

10. The mixture as claimed in claim 8 or 9, **characterized in that** said vitamin P contains 60 - 80% of total flavonoids, 12 - 30% of anthocyanin and 3 - 20% of catechin.
